**Europäisches Patentamt**

**European Patent Office**

**Office européen des brevets**

(19)

(11) Publication number : **0 417 571 B1**

# (12) EUROPEAN PATENT SPECIFICATION

(45) Date of publication of patent specification :
24.05.95 Bulletin 95/21

(51) Int. Cl.⁶ : **G21C 17/022, G01N 27/30**

(21) Application number : **90116753.6**

(22) Date of filing : **31.08.90**

(54) **System and electrode for "in situ" monitoring the quality of high temperature water in power plants.**

(30) Priority : **11.09.89 JP 232824/89**

(43) Date of publication of application :
**20.03.91 Bulletin 91/12**

(45) Publication of the grant of the patent :
**24.05.95 Bulletin 95/21**

(84) Designated Contracting States :
**DE SE**

(56) References cited :
EP-A- 0 160 566
EP-A- 0 220 666
EP-A- 0 313 657
LU-A- 79 771
INTERNATIONAL SYMPOSIUM-ON SAFETY
ASPECTS OF THE AGEING AND MAINTE-
NANCE OF NUCLEAR POWER PLANTS. VIEN-
NA,29/06-03/07 1987 -
IAEA-SM-295/11;Aaltonen et al.: WATER
CHEMISTRY MONITORING IN PREVENTION
OF AGEING RELATED CORROSION
PHENOMENA. Par. 3-4

(73) Proprietor : **HITACHI, LTD.**
**6, Kanda Surugadai 4-chome**
**Chiyoda-ku, Tokyo 101 (JP)**

(72) Inventor : **Sakai, Masanori**
**1-19-4-102, Ishinazaka-cho**
**Hitachi-shi, Ibaraki-ken (JP)**
Inventor : **Mabuchi, Katsumi**
**1-6-3-404, Nishinarusawa-cho**
**Hitachi-shi, Ibaraki-ken (JP)**

Inventor : **Arato, Toshiaki**
**6-1-302, Ishikawa-cho**
**Katsuta-shi, Ibaraki-ken (JP)**
Inventor : **Takahashi, Takuya**
**1-32-6-402, Nishinarusawa-cho**
**Hitachi-shi, Ibaraki-ken (JP)**
Inventor : **Izumiya, Masakiyo**
**2531-2, Motoyoshidacho**
**Mito-shi, Ibaraki-ken (JP)**
Inventor : **Masaoka, Isao**
**4-17-12, Nishinarusawa-cho**
**Hitachi-shi, Ibaraki-ken (JP)**
Inventor : **Kojima, Yoshitaka**
**5-39-3, Kuji-cho**
**Hitachi-shi, Ibaraki-ken (JP)**
Inventor : **Inagaki, Masahisa**
**1262-21, Ishinazaka-cho**
**Hitachi-shi, Ibaraki-ken (JP)**
Inventor : **Ohsumi, Katsumi**
**2-21-10, Hanayama-cho**
**Hitachi-shi, Ibaraki-ken (JP)**
Inventor : **Hayashi, Makoto**
**4-3-35, Moriyama-cho**
**Hitachi-shi, Ibaraki-ken (JP)**
Inventor : **Sato, Fumio**
**2-8-1, Hanayama-cho**
**Hitachi-shi, Ibaraki-ken (JP)**
Inventor : **Suwa, Masateru**
**1220-191, Muramatsu,**
**Tohkai-mura**
**Naka-gun, Ibaraki-ken (JP)**
Inventor : **Akahori, Kimihiko**
**3-12-10, Sasano-cho**
**Kasuta-shi, Ibaraki-ken (JP)**

(74) Representative : **Patentanwälte Beetz - Timpe -**
**Siegfried Schmitt-Fumian - Mayr**
**Steinsdorfstrasse 10**
**D-80538 München (DE)**

## Description

The present invention relates to a system and electrodes for monitoring chemical properties of high temperature water in power plants such as nuclear and thermal power plants whereby through in-situ sampling and diagnosis a high reliability is achieved.

JP-A-58-51312 (1983) and US-A-4 552 718 disclose monitoring devices for monitoring the plant operation.

However, in JP-A-58-51312 there is no disclosure regarding a method of obtaining information about the water quality or a method of water quality evaluation and no disclosure regarding a monitoring and controlling system in which information from water quality sensors is evaluated, and the water quality is monitored and controlled. Further, US-A-4 552 718 discloses in detail a method and a system for plant operation control with regard to the operation status (output amount, cooling) water level, temperature reactor, etc.,) of a nuclear power plant. However, it does not disclose a water quality diagnosis and control system and technology such as a method for sensing the water quality itself, nor an evaluation method and a display system.

JP-A-60-183595 (1985) discloses a method wherein the pH of a nuclear reactor cooling water is detected and adjusted within a range between 7.0 and 8.5, but does neither specify a technology with regard to a pH sensor, nor clarify the relationship between the pH and other water quality information, and further does not specify a method for displaying during control.

ECP sensors (reference electrodes or corrosion potential sensors) in a high temperature and high pressure water are discussed in "Reference Electrode for PWR, Copyright 1987, Electric Power Research Institute, Inc." which is a comprehensive paper dealing with reference electrodes in high temperature and high pressure water of recent years. The Ag/AgCl (silver/silver chloride) ECP sensor which is employed according to this paper has a structure wherein the silver/silver chloride regardless to an inner or an outer reference electrode is immersed in a solution including $Cl^-$ ions.

The Ag/AgCl electrodes are fundamental and representative reference electrodes for electrochemical measurements, and these electrodes have a small temperature coefficient and have been used for high temperature and high pressure water measurements until now. However, under severe environmental conditions such as high temperature and high pressure water there is the problem that the sensor is deteriorated by potential variations due to the leakage of $Cl^-$ ions accompanied with a deterioration of the water seal in particular; accordingly, there were no ECP sensors which were disposed in a nuclear reactor and had a life time level enduring the periodic inspection intervals.

A hydrogen ion concentration sensor for use high temperature and high pressure water is discussed in "Journal of Electrochemical Society, 132, 1866 (1985) p15-42". The problem herein is low to seal it, or how to improve the durability of the sensor itself during its use in the reactor. Since the pH is measured without use of a liquid junction, and the sensor is isolated from the high temperature environment through a ceramic cylinder (the porosity of which is near zero), and use is made of the semiconductor property of the ceramic, its impedance becomes extremely high, and there remain problems such as a countermeasure to noise and a method of isolation between the cylinder interior and the high temperature water environment.

With regard to a method for the evaluation of measurement results and display, the above JP-A- 58-51312 also discloses a measure for displaying a plant status during plant operation such as nuclear reactor pressure and cooling water level on the CRT of a computer as a specific screen image, however, in this case again no technology with regard to sensing the respective states and to monitoring the water quality information is referred to, and further no indication is made which water quality informations are to be combined, and how these are to be evaluated.

Further, the sensor technology was not taken into consideration when controlling the water quality by monitoring the water quality and feeding back the water quality data to the control system, so that there was the actual problem that the water quality monitoring and control system itself was impracticable due to the lacking reliability of the obtained data and the insufficient durability of the sensors. Therefore, because no reliable sensors were available, the system was limited to plant operation control based on the mechanical aspects and the status of the plant, and no integral consideration was made regarding an evaluation method based on a technology which measures and controls the very water quality factors, and based on a monitoring data display system and a displayed screen image, and other status criteria, such as an abnormal sound of a pump and the water quality status near the pump, the temperature and the results of water quality monitoring data. Accordingly, there was the technical problem that the plant could not be operated and controlled with the necessary and sufficient information.

In JP-A- 58-215549 (1984) there is disclosed an electrochemical sensor partitioned by a diaphragm and filled with electrolyte. However, for use in such environments as nuclear or thermal power plants where a stringent quality control of the high temperature water is necessary, the heat resistance and the durability of the sensor are not satisfactory for these practical applications.

JP-A- 58-215549 further discloses that means for separation and determination of dissolved hydrogen may be effective. In this case, however, because a selective hydrogen-permeable membrane is required, there will still arise the like problem.

In "Electrochemical Methods", John Wiley & Sons, Inc. (1980) pp.553-573, an electrochemical measurement apparatus for use in nuclear power plants is described. However, there is no description on an electrochemical cell capable of being installed incore of a nuclear plant or in a piping system of a plant, and further having a low impedance reference electrode characteristic.

EP-A-220 666 relates to a system for automatically sampling, monitoring, analyzing and adjusting the quality of power plant steam cycle water. In addition to the determination of the specific conductivity and the pH, the monitoring of the sodium content, the dissolved oxygen content, the hydrazine content and the ammonia content may be taken from Fig. 2 of this document, which, however, is silent about how these analysis may be made. It specifically relates to the mathematical determination of the specific conductivity on the basis of a chromatographic determination of the concentration of those chemical species for which no simple in-line monitoring is available, e.g. for organic acids. Further, this prior art system is based on the analysis of water samples taken out from the system to be analyzed, and not on in-situ measurements.

US-A-4 764 338 disclosed a method for operating BWR power plants wherein the free $^{90}$Co concentration in the core water is controlled by adjusting the pH value to 7.0 to 8.5. This document does not relate to in-situ monitoring of the water quality.

The congress paper of P. Aaltonen and I. Aho-Mantila "Water chemistry monitoring in prevention of ageing related corrosion phenomena", presented at the International symposium on safety aspects of the ageing and maintenance of nuclear power plants, IAEA-SM-295/11, Vienna, Austria, 29 June - 3 July 1987, pages 1-20, deals, inter alia, with the problems encountered with a direct in-situ measurement of power plant water properties by means of electrochemical sensors, however, is quite silent about any concrete realization of such a system.

EP-A-0 313 657 discloses a miniature reference electrode for use in vivo or in body fluids on the basis of a (Pt or Ag)/AgX,Ag$_2$O system wherein a porous ceramic member is provided on the electrolyte. This porous member, which may be a ceramic, comprises an ion-permeable portion having a predetermined diffusion coefficient. The problem of high temperature measurements is not mentioned in this document.

The objects of this invention are to provide a monitoring system and an electrode for monitoring chemical properties of high temperature water in power plants such as nuclear or thermal power plants with high precision by means of in-situ measurement. The system should be suited to provide precise and adequate information directly relating to the water quality from a plant in operation. It should further be capable of controlling the water quality specifically by means of predicting status changes in a plant from a comparison between existing water information and accumulated monitoring data.

The above objects are achieved according to the independent claims 1 and 8. The dependent claims relate to preferred embodiments.

The monitoring system of the present invention for monitoring chemical properties of high temperature water in power plants comprises

- an electrochemical sensor being in electrochemical contact with the water for detecting the respective water property,
- means receiving the information sampled from the sensor for analyzing the respective wtare property on the basis of the sampled information and for comparing the obtained results with predetermined reference values for the plant operation and manipulation, and
- means for displaying and/or recording necessary portions of the comparison results; it is characterized in that
- the electrochemical sensor is disposed in direct contact with the high temperature water, and
- the electrochemical sensor is in electrochemical contact with the water through a porous material which substantially suppresses dissolution of the sensor electrolyte into the high temperature water, yet being ion-permeable.

The electrochemical electrode of the present invention for detecting electrochemical properties of solutions comprises

- an electrode member which is in electrochemical contact with an electrolyte,
- a terminal for electrically contacting the electrode member, and
- a non-liquid-permeable porous ceramic or resin material disposed on the electrolyte; it is characterized in that a further porous material is provided on the porous material disposed on the electrolyte.

In the following, the invention will be described with reference to preferred embodiments in connection with

EP 0 417 571 B1

the accompanying drawings.

Fig.1 is a schematic representation illustrating a plant operating condition monitoring system for a nuclear power plant in which the present invention is employed;

Fig.2 is a cross-sectional view illustrating one structure of a halogen-containing inner reference electrode;

Fig.3 is a graph illustrating the change of the corrosion potential of a structural material in the reactor with time, and the hydrogen gas injection effect;

Fig.4 is a graph illustrating the potential difference between the halogen-containing inner reference electrodes;

Fig.5 is a graph illustrating the pH change in the reactor with time, and the alkali injection effect;

Fig.6 is a cross-sectional view illustrating another structure of halogen-containing inner reference electrode;

Fig.7 is a graph illustrating the change of the corrosion potential of a structural material in the reactor with time, and the chemical injection effect;

Fig.8 is a graph illustrating the change of the corrosion potential of a structural material in the reactor and the change of the dissolved oxygen concentration with time, and the hydrogen gas injection effect;

Fi.9 is a graph illustrating the change of the corrosion potential of a structural material in the reactor and the change of the dissolved hydrogen peroxide concentration with time, and the chemical injection effect;

Fig.10 is a graph illustrating the change of the corrosion potential of a structural material in the reactor and the change of the dissolved oxygen, hydrogen, and hydrogen peroxide concentrations with time, and the chemical injection effect;

Fig.11 is a graph illustrating the change of the corrosion potential of a crack sensor and the change of the crack developing amount of the sensor with time, and the chemical injection effect;

Fig.12 is a partial cross-sectional view illustrating the structure of a nuclear fuel assembly where the present invention is applied;

Fig.13 is a graph illustrating the relationship between the corrosion potential of a fuel cladding tube made of a Zr alloy and the thickness of the oxide coating film of the Zr alloy, and

Fig.14 and Fig.15 are flowcharts illustrating the plant operating conditions monitoring system of the present invention;

Fig.16 is a cross-sectional structure of an electrochemical sensor comprising an internal reference electrode, a working electrode, a counterelectrode, and a pH electrode;

Fig.17 is a detailed cross-sectional view of a ceramic silver/silver ion internal reference electrode;

Fig.18 (a) shows a current (i)-potential (E) curve for a system of a three-species mixture of oxygen, hydrogen peroxide and hydrogen;

Fig.18 (b) is a diagram illustrating random pulse signals applied to a working electrode in an electrochemical sensor;

Fig.19 illustrates the separation and determination of dissolved oxygen, hydrogen peroxide and hydrogen by means of a random pulse method, in relation to the time dependence, and the effect of a hydrogen injection;

Fig.20 is a diagram illustrating the structure of the neural network used in determining scalar fields.

In accordance with the present invention, information is consecutively sampled directly relating to the water quality of an objective section of the monitoring plant in a predetermined period through the electrochemical sensor disposed in said objective monitoring section of the plant.

The above electrochemical sensor comprises a porous material which permits electrochemical contact between the surrounding high temperature water and the electrolyte of the sensor but suppresses an excessive dissolution of the electrolyte into the high temperature water. The porous ceramic or resin covering material is non-liquid-permeable but ion-permeable. That is, the covering material prevents liquid from going through the material into the electrolyte, but allows the liquid to be in electrochemical contact with the electrolyte. Only ions of the electrolyte can permeate through the covering material.

In accordance with the present invention, a reference electrode (ECP sensor) and a pH sensor (hydrogen ion concentration meter) with high durability, high reliability and practical applicability, and an electrochemical cell or sensor capable of determining dissolved oxygen, hydrogen peroxide and the like by using the reference electrode thereof are provided. The system may furnish various water quality data (pH, corrosion potential, dissolved oxygen, dissolved hydrogen, dissolved hydrogen peroxide, electrical conductivity of solution, etc.) based on the electrochemical measurement, and may comprise a crack sensor.

The structure of the electrochemical reference electrode of the present invention is characterized by comprising an electrolyte layer containing ions of the electrode material, a porous ceramic layer surrounding the electrolyte layer preventing liquid permeation, an electrode member being in electrochemical contact with the electrolyte, and a terminal for electrically contacting the electrode member. By this structure, the impedance

4

of the reference electrode has been successfully reduced low enough to be suited for an improved electrochemical cell or electrochemical sensor comprising a three-electrode system of a working electrode, a counterelectrode and a reference electrode, or a two-electrode system thereof.

Further, a heat resistant porous jacket layer surrounding the ceramic layer and preventing liquid permeation is provided. The electrochemical reference electrode may further comprise an electrolyte layer composed of a halide of the electrode material.

It may further comprise a sheath protecting the porous jacket layer and having part of the same exposed therethrough allowing part of the layer to contact with water to measure.

According to an exemplary embodiment, the reference electrode comprises a solid electrolyte layer containing positive ion and negative ion of the electrode member the inner porous material is a ceramic layer surrounding the same, and the further porous material is a heat resistant ceramic material. As the solid electrolyte layer, a combination of an oxide of the electrode metal member/metal as well as a metal halide/metal may be used.

The invention allows the water quality diagnosis and the water quality control by means of the compact ceramic-coated type sensor of adjustable porosity with sufficient accuracy and sensor durability in-situ in a more specific and systematic manner.

The water quality diagnosis data which are monitored by an arithmetic processing unit and displayed on a CRT display adequately indicate water quality information obtained through the ceramic-coated or resin-coated type sensor system and do not provide erroneous information. Thus, operators are enabled to determine and adjust the water quality of the plant by immediately feeding back the results obtained from the water quality display system to the plant water quality operation control.

The establishment of the water quality information acquiring technology with adequate and high accuracy from the sensor system allows a determination of the mutual relationship of data, a corresponding display and the determination of the evaluation standard, thus allowing plant operation and water quality control with a higher standard.

The single or plural sensors have a structure in which electrodes of silver/silver halide, silver sulfate or single silver electrodes are tightly overcoated with press-formed ceramics or microscopic resin powders, or are directly coated at their surface with a ceramic material having a solution impregnatable aperture ratio, obtained through measures such as a direct plasma spray, electrophoresis, combination of the both, etc., so that because of the non-occurence of halide ions in the solution the present sensor may be used for indicating the potential of the standard electrode (reference electrode or ECP sensor).

Further, such a sensor may be used as pH sensor in which silver/silver oxide, platinum/thallic oxides ($Tl_2O_3$), mercury/mercury oxide, copper/copper oxide or indium/$Ir_2O_3$ are provided and directly coated by ceramics or by press-formed ceramics or microscopic resin powders. This sensor responds by a potential corresponding to the pH value. In principle, the pH sensor may be used as ECP sensor when the pH conditions are constant, or the variation of the pH is negligible. Disposition of both the above ECP sensor and the pH sensor is practical.

It is preferable to dispose either the ECP sensor or the pH sensor or both or a plurality thereof in the vicinity of a nuclear reactor core, and instrumentation piping, a separator or its vicinity, a mixing plenum or its vicinity, a downcomer or its vicinity, a jet pump or its vicinity, a downstream plenum chamber or its vicinity, or a cooling water piping line.

In the piping line of the reactor means are disposed for controlling the water quality by injecting a gas such as oxygen and hydrogen or chemicals.

A measuring system for electrochemically analyzing dissolved chemical species existing in a solution may be provided which respectively uses at least one of a three-electrode system composed of one working electrode, a reference electrode (ECP sensor) and a counterelectrode and a two-electrode system employing two electrodes of the three.

The water quality analysis, the data reading of the analyzed results and the display are carried out by using an arithmetic processing unit, a printer, a plotter, and a cathode ray tube display system (CRT). The water quality sensors in toto seven kinds, include crack sensors (M), sensors of dissolved hydrogen (DH), dissolved oxygen (DO), the pH value (pH), the electrochemical potential (ECP), the electric conductivity (S) of the solution and sensors for dissolved hydrogen peroxide ($DH_2O_2$); the are disposed while combining them properly. The water quality diagnosis control is carried out by selecting water quality diagnosis data from two kinds of sensors among the seven sensors and comparing and examining with a standard value put in beforehand based on the display of the CRT display system, the printer, the plotter and the recorder.

The water quality diagnosis control can be carried out based on the display of the CRT, the printer, the plotter and the recorder using DO and ECP. The water quality analysis, the data reading of the analyzed results and the display are carried out by using an arithmetic processing unit, a printer, a plotter, and a CRT display system.

A plurality of sensors such as the crack sensor (M) measuring the crack development amount, the dissolved hydrogen sensor (DH), the dissolved oxygen sensor (DO), the corrosion potential or the reference electrode (ECP sensor), the electric conductivity meter (S) and the dissolved hydrogen peroxide ($DH_2O_2$), may be used in combination for water quality determination.

Several examples of sensor combinations and their combination with the display measures are explained below:

(1) Water quality diagnosis and control are carried out based on the display of the CRT, the printer, the plotter and the recorder and on DH and DO.

(2) Water quality diagnosis and control are carried out based on the display of the CRT, the printer, the plotter and the recorder and on DH and pH.

(3) Water quality diagnosis and control are carried out based on the display of the CRT, the printer, the plotter and the recorder and on DH and S.

(4) Water quality diagnosis and control are carried out based on the display of the CRT, the printer, the plotter and the recorder and on DH and M.

(5) Water quality diagnosis and control are carried out based on the display of the CRT, the printer, the plotter and the recorder and on DH and $DH_2O_2$.

(6) Water quality diagnosis and control are carried out based on the display of the CRT, the printer, the plotter and the recorder and on DH and pH.

(7) Water quality diagnosis and control are carried out based on the display of the CRT, the printer, the plotter and the recorder and on DO and S.

(8) Water quality diagnosis and control are carried out based on the display of the CRT, the printer, the plotter and the recorder and on DO and M.

(9) Water quality diagnosis and control are carried out based on the the display of the CRT, the printer, the plotter and the recorder and on DO and $DH_2O_2$.

(10) Water quality diagnosis and control are carried out based on the display of the CRT, the printer, the plotter and the recorder and on DO and ECP.

(11) Water quality diagnosis and control are carried out based on the display of the CRT, the printer, the plotter and the recorder and on pH and ECP.

(12) Water quality diagnosis and control are carried out based on the display of the CRT, the printer, the plotter and the recorder and on pH and S.

(13) Water quality diagnosis and control are carried out based on the display of the CRT, the printer, the plotter and the recorder and on pH and M.

(14) Water quality diagnosis and control are carried out based on the display of the CRT, the printer, the plotter and the recorder and on pH and $DH_2O_2$.

(15) Water quality diagnosis and control are carried out based on the display of the CRT, the printer, the plotter and the recorder and on ECP and S.

(16) Water quality diagnosis and control are carried out based on the display of the CRT, the printer, the plotter and the recorder and on ECP and M.

(17) Water quality diagnosis and control are carried out based on the display of the CRT, the printer, the plotter and the recorder and on ECP and $DH_2O_2$.

(18) Water quality diagnosis and control are carried out based on the display of the CRT, the printer, the plotter and the recorder and on S and M.

(19) Water quality diagnosis and control are carried out based on the display of the CRT, the printer, the plotter and the recorder and on S and $DH_2O_2$.

(20) Water quality diagnosis and control are carried out based on the display of the CRT, the printer, the plotter and the recorder and on M and $DH_2O_2$.

(21) Water quality diagnosis and control are carried out based on the display of the CRT, the printer, the plotter and the recorder and on DH), DO) and pH.

(22) Water quality diagnosis and control are carried out based on the display of the CRT, the printer, the plotter and the recorder and on DH, DO and ECP.

(23) Water quality diagnosis and control are carried out based on the display of the CRT, the printer, the plotter and the recorder and on DH, DO and S.

(24) Water quality diagnosis and control are carried out based on the display of the CRT, the printer, the plotter and the recorder and on DH, DO and M.

(25) Water quality diagnosis and control are carried out based on the display of the CRT, the printer, the plotter and the recorder and on DH, DO and $DH_2O_2$.

(26) Water quality diagnosis and control are carried out based on the display of the CRT, the printer, the plotter and the recorder and on DH, pH and ECP.

(27) Water quality diagnosis and control are carried out based on the display of the CRT, the printer, the plotter and the recorder and on DH, pH and S.

(28) Water quality diagnosis and control are carried out based on the display of the CRT, the printer, the plotter and the recorder and on DH, pH and M.

(29) Water quality diagnosis and control are carried out based on the display of the CRT, the printer, the plotter and the recorder and on DH, pH and $DH_2O_2$.

(30) Water quality diagnosis and control are carried out based on the display of the CRT, the printer, the plotter and the recorder and on DH, ECP and S.

(32) Water quality diagnosis and control are carried out based on the display of the CRT, the printer, the plotter and the recorder and on DH, ECP and $DH_2O_2$.

(33) Water quality diagnosis and control are carried out based on the display of the CRT, the printer, the plotter and the recorder and on DH, S and M.

(34) Water quality diagnosis and control are carried out based on the display of the CRT, the printer, the plotter and the recorder and on DH, S and $DH_2O_2$.

(35) Water quality diagnosis and control are carried out based on the display of the CRT, the printer, the plotter and the recorder and on DO, pH and ECP.

(36) Water quality diagnosis and control are carried out based on the display of the CRT, the printer, the plotter and the recorder and on DO, pH and S.

(37) Water quality diagnosis and control are carried out based on the display of the CRT, the printer, the plotter and the recorder and on DO, pH and M.

(38) Water quality diagnosis and control are carried out based on the display of the CRT, the printer, the plotter and the recorder and on DO, pH and $DH_2O_2$.

(39) Water quality diagnosis and control are carried out based on the display of the CRT, the printer, the plotter and the recorder and on DO, ECP and S.

(40) Water quality diagnosis and control are carried out based on the display of the CRT, the printer, the plotter and the recorder and on DO, ECP and M.

(41) Water quality diagnosis and control are carried out based on the display of the CRT, the printer, the plotter and the recorder and on DO, ECP and $DH_2O_2$.

(42) Water quality diagnosis and control are carried out based on the display of the CRT, the printer, the plotter and the recorder and on DO, S and M.

(43) Water quality diagnosis and control are carried out based on the display of the CRT, the printer, the plotter and the recorder and on DO, S and $DH_2O_2$.

(44) Water quality diagnosis and control are carried out based on the display of the CRT, the printer, the plotter and the recorder and on pH, ECP and S.

(45) Water quality diagnosis and control are carried out based on the display of the CRT, the printer, the plotter and the recorder and on pH, ECP and M.

(46) Water quality diagnosis and control are carried out based on the display of the CRT, the printer, the plotter and the recorder and on pH, ECP and $DH_2O_2$.

(47) Water quality diagnosis and control are carried out based on the display of the CRT, the printer, the plotter and the recorder and on pH, S and M.

(48) Water quality diagnosis and control are carried out based on the display of the CRT, the printer, the plotter and the recorder and on pH, M and $DH_2O_2$.

(49) Water quality diagnosis control is carried out based on the display of the CRT, the printer, the plotter and the recorder and on ECP, S and M.

(50) Water quality diagnosis and control are carried out based on the display of the CRT, the printer, the plotter and the recorder and on ECP, S and $DH_2O_2$.

(51) Water quality diagnosis and control are carried out based on the display of the CRT, the printer, the plotter and the recorder and on S, M and $DH_2O_2$.

(52) Water quality diagnosis and control are carried out based on the display of the CRT, the printer, the plotter and the recorder and on the time-based change of DH in the water quality analysis results.

(53) Water quality diagnosis and control are carried out based on the display of the CRT, the printer, the plotter and the recorder and on the time-based change of DO in the water quality analysis results.

(54) Water quality diagnosis and control are carried out based on the display of the CRT, the printer, the plotter and the recorder and on the time-based change of pH in the water quality analysis results.

(55) Water quality diagnosis and control are carried out based on the display of the CRT, the printer, the plotter and the recorder and on the time-based change of ECP.

(56) Water quality diagnosis and control are carried out based on the display of the CRT, the printer, the plotter and the recorder and on the time-based change of S in the water system.

(57) water quality diagnosis and control are carried out based on the display of the CRT, the printer, the plotter and the recorder and on the time-based change of the measured results M of the ECP sensor.

(58) Water quality diagnosis and control are carried out based on the display of the CRT, the printer, the plotter and the recorder and on the time-based change of $DH_2O_2$ in the water quality analysis results.

Water quality diagnosis and control may be carried out based on the display of the CRT, the printer plotter and the recorder and on respective time-based changes of two kinds of analyzed data. Further, water quality diagnosis and control can be carried out based on the display of the CRT, the printer, the recorder and on respective time-based changes of three kinds of analyzed data.

In the water quality diagnosis, the water quality is analyzed using a random pulse voltammetry or plural kinds of voltametries in an electrochemical cell of this invention. With the random voltammetry, a data compression method and a neural network are used. Among the plural kinds of voltammetries, either of the five kinds of pulse voltammetry may be used, i.e., reverse pulse, normal pulse, differential pulse, square wave and normal differential pulse voltammetry. Water quality is controlled by using plural kinds of voltammetries or the random pulse voltammetry based on the above pattern recognition and simultaneously identifying $O_2$, $H_2$ and $H_2O_2$. Further, analyzed results such as of $SO_4{}^{2-}$, $Cl^-$ and $Na^+$ which are decomposition products of core purification resins are also displayed and compared with standard values putin in advance.

There is a function for controlling the injection amount of $N_2H_4$ (hydrazin) into a steam generator based on the dissolved $O_2$ gas data in a nuclear reactor primary cooling water line. The injection amount of $H_2$ gas is controlled based on dissolved $H_2O_2$ data in the nuclear reactor primary cooling water line. The injection amount of $H_2$ gas may be controlled based on the measured data of $H_2$, $O_2$ and $H_2O_2$. The injection amount of $H_2$ gas is controlled based on the dissolved $H_2$ amount in the nuclear reactor primary cooling water line. The injection amount of $H_2$ into the nuclear reactor primary cooling water is controlled based on the dissolved $O_2$ gas amount in the nuclear reactor primary cooling water line. Further, the injection amount of $H_2$ gas into the nuclear reactor primary cooling water is controlled based on dissolved $H_2O_2$ in the nuclear reactor primary cooling water. The corrosion amount may be monitored by detecting the potential of a Zr alloy member of the nuclear fuel assembly in a nuclear power plant.

Embodiment 1

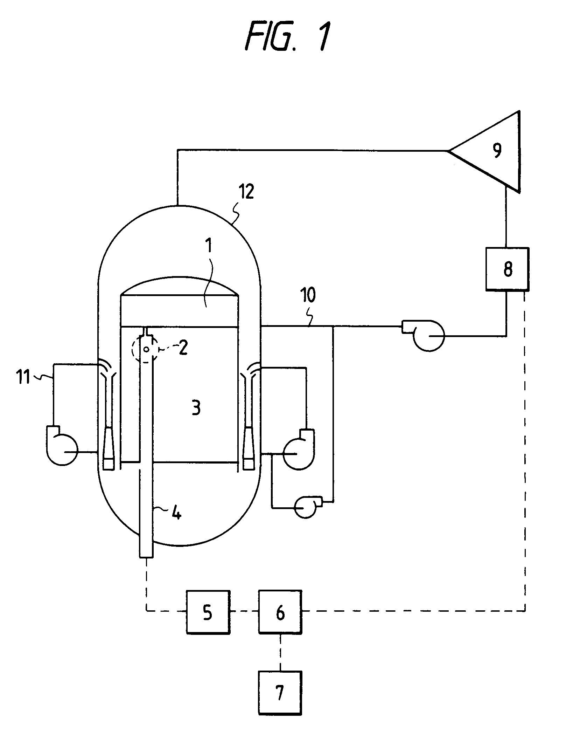

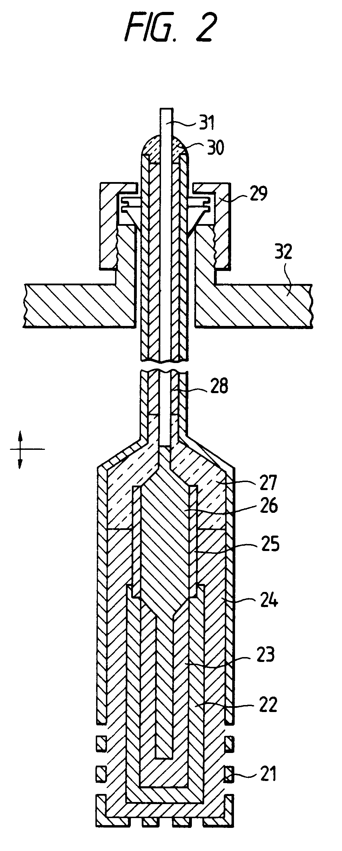

The present embodiment is an example wherein the water quality control in a nuclear reactor was tried by disposing the ECP sensor as shown in Fig.2 in the nuclear reactor of a BWR plant as shown in Fig.1 through an instrumentation piping and by measuring the time-based change in the corrosion potential of the reactor structural material under this water quality environment with reference to the potential of the ECP sensor (corresponding to the reaction of AgCl + e → Ag + $Cl^-$ etc.)

In Fig.1, the ECP sensor 2 is inserted in a reactor instrumentation piping 4 of the core 3 in the nuclear reactor pressure vessel 12. 1 is a drier, 5 is a potentiostat of the electrochemical interface, 6 is a computerized arithmetic processing automatic control unit, 7 is a remote control command unit and an analysis result display and output unit, 8 is a gas and chemical injection line, 9 is a turbine, 10 is a nuclear reactor water supply pipe, and 11 is a nuclear reactor water recirculation line.

Fig.2 shows the constitution of an ECP sensor according to the present invention. The present silver/silver halide electrode does not at all include a solution layer containing an electrolyte such as KCl as its constitutional element.

In the drawing, a part of a silver wire 26 is coated with alumina or zirconia through a CVD method, and on the portion not coated with the ceramic material, silver halide (AgCl, AgBr or AgI) 23 is adhered. After provision of the silver halide, an alumina coating 22 having a thickness of several hundreds of μm and porosity ratio of below 70% is formed on the surface through an electrophoresis method. Further, around its periphery an alumina coating 24 having a thickness of a few mm and a porosity ratio of below 10% is formed through a plasma spray method or by compression forming. If no silver halide is used, a structure of a silver wire 26 directly coated with ceramics layers 24 and 25 is able to function as an ECP sensor of $Ag/Ag^+$.

The sensor portion thus constructed is enclosed by a SUS316L guard 21 provided with a small aperture at its bottom to safeguard the sensor portion. The free end of the silver wire 26 is electrically connected to a lead wire 31 made of SUS316L. The top of the sensor portion is sealed with glass 27. Further, the space between the guard 21 and the lead wire 31 is filled with magnesia 28 to isolate the both, and the ends thereof are sealed with glass 30.

This sensor is fixed at a fixing member 29 in the instrumentation piping 4, and the sensor signals are taken out to the outside through a cable 32. With the sensor thus constituted, the high temperature water of the plant is not in direct contact with the solid electrolyte (silver halide) so that an extremely long lifetime is achieved.

Accordingly, the sensor is disposed in the high temperature water of the plant during the period from one inspection to the next inspection of the plant, and direct data with respect to the water quality are continuously obtained so that the reliability of the data is extremely increased.

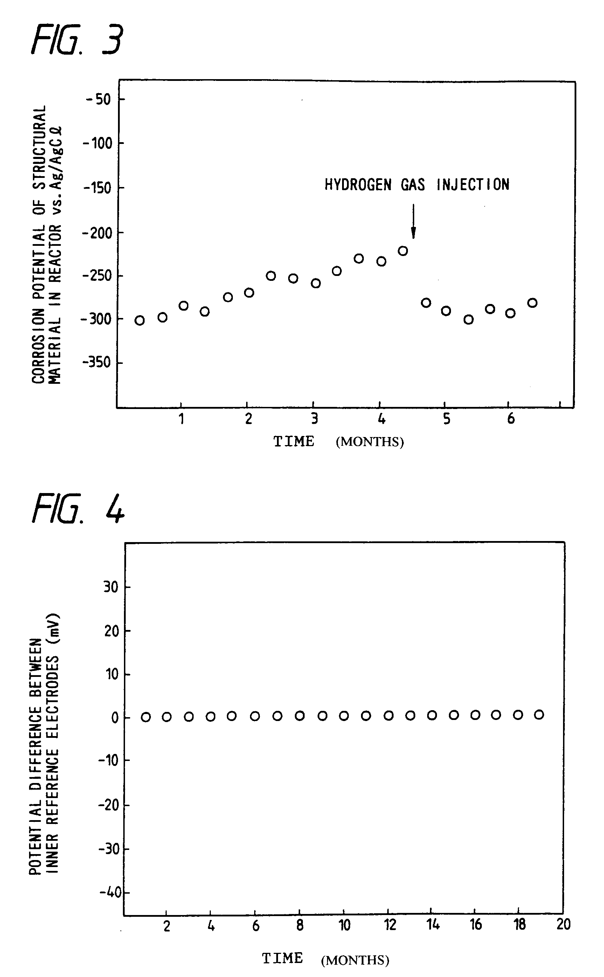

Fig.3 illustrates the time dependence of the corrosion potential of the reactor structural member by using the ECP sensor the output of which is supplied to the analysis result display system (cathode ray tube display system) 7 of Fig.1. When hydrogen gas is injected from the gas injection line 8, the corrosion potential of the reactor structural material decreases with good response which proves that the water quality diagnosis and control system is sufficiently useful for the corrosion suppression.

As shown in Fig.4, no potential difference to another identical ECP sensor installed in the reactor instrumentation piping 4 appears on the displays system 7 for a long time, which proves that this ECP meter has an excellent stability and durability.

Embodiment 2

This embodiment is an example wherein a pH meter has been installed in the reactor intrumentation piping 4 shown in Fig.1. By measuring the potential of this pH meter in comparison with the potential of the ECP sensor providing the reference potential shown in Fig.2, a potential output is obtained corresponding to the hydrogen ion concentration of the solution. The constitution of the pH meter used here is the same as that shown in Fig.2 except that $Pt/Tl_2O_3$, $Ag/Ag_2O$, $Hg/Hg_2O$, $Cu/Cu_2O$, $Cu/CuO$, $Ir/Ir_2O_3$ or $Ir/IrO_2$ are used in place of the silver halide in Fig.2.

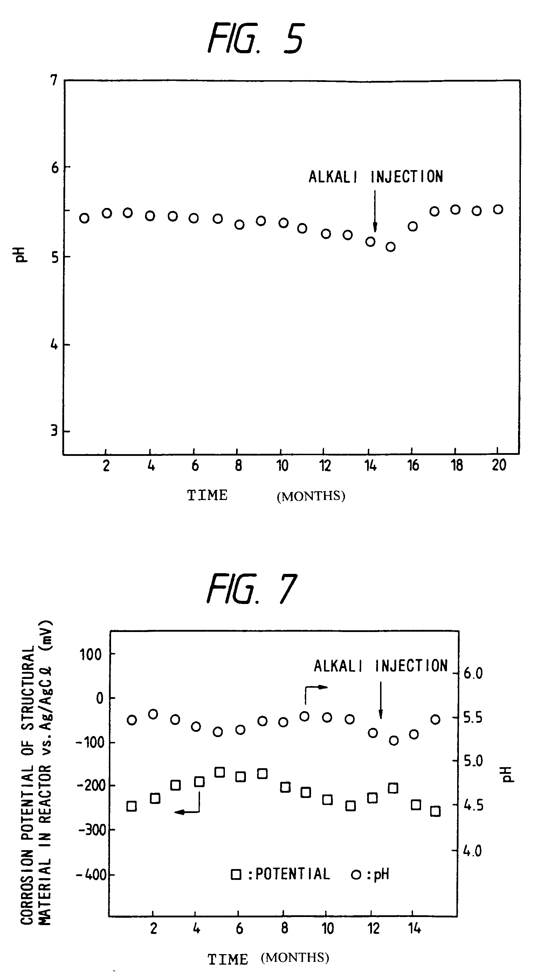

Fig.5 shows the time-based change of the pH (negative logarithm of hydrogen ion concentration). When a slight amount of alkali is injected from the injection system 8 during a slight fall of pH in Fig.5 displayed on the display system, the pH in the reactor begins to rise after a predetermined interval which shows that the water quality diagnosis and control are carried out satisfactorily.

Embodiment 3

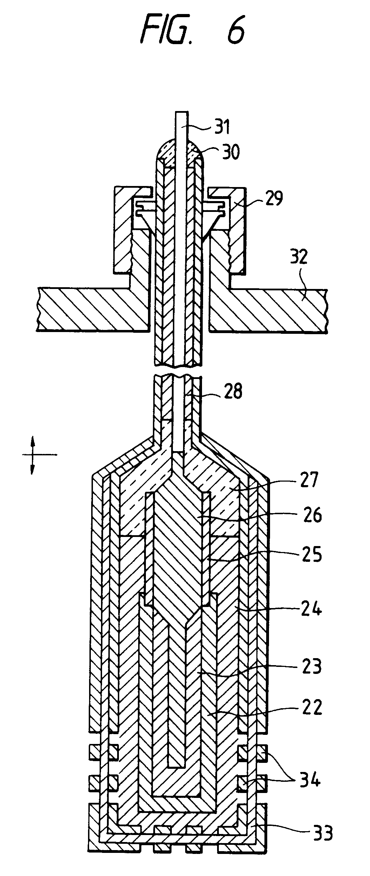

In this embodiment like in embodiment 2 the water quality environment in the reactor is monitored by simultaneously inserting an inner reference electrode and a pH sensor in the reactor instrumentation piping 4. The structure of the inner reference electrode used here is fundamentally the same as that shown in Fig.2 except that the ceramic layer of the SUS316L guard and its inner liner is double-structured. Its structure is shown in Fig.6. In this embodiment, the difference to Fig.2 is that the coating such as alumina and zirconia is sandwiched by the SUS316L guard. Further, the pH electrode used in this embodiment also uses the double-structured guard as shown in Fig.6.

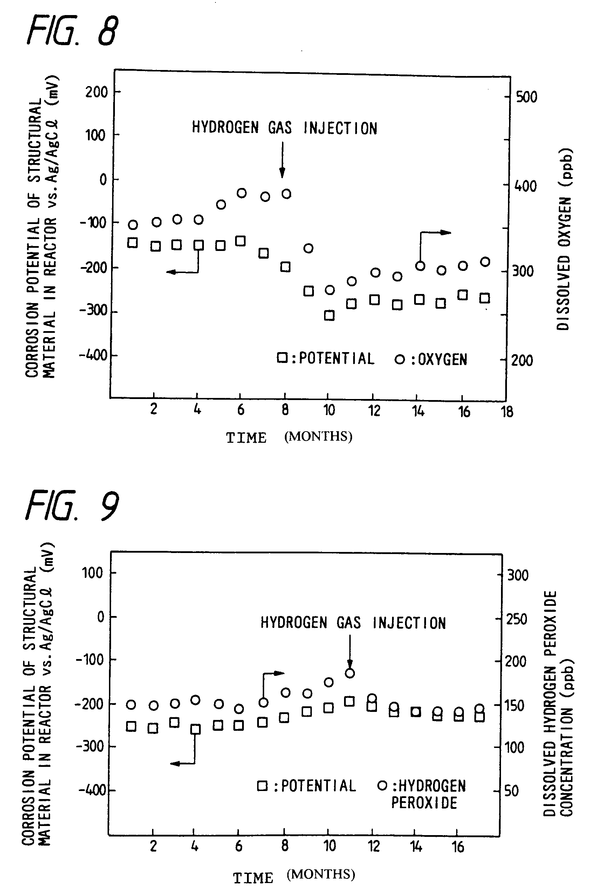

On the display system 7 the relationship between the corrosion potential of the reactor structural material in Fig.7 and the pH is displayed, and generally there appears a tendency of the corrosion potential of the structural material to rise with falling pH.

When alkali is injected from the chemical injection system 8, there appears a clear tendency of falling of the corrosion potential with rising pH so that a sufficient increase in corrosion resistance of the reactor structural material is effected through the water quality diagnosis and control.

Embodiment 4

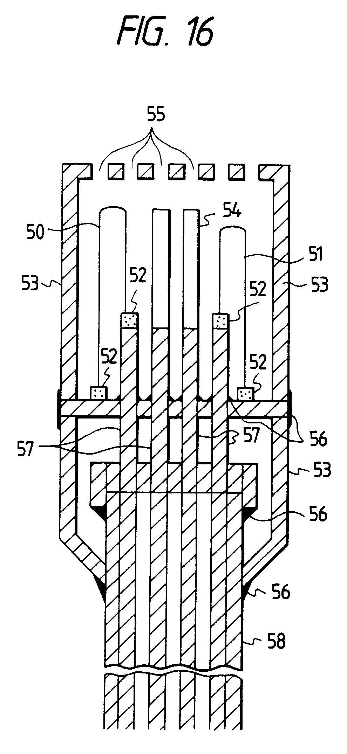

In this embodiment, an electrochemical measuring system comprising an integrated platinum microelectrode 50 as shown in Fig.16 as sensor of dissolved oxygen, hydrogen and hydrogen peroxide and the inner reference electrode as shown in Fig.2 and Fig.6 are installed in the reactor instrumentation piping 4. For obtaining the results of Fig.8, the platinum microelectrode was used as dissolved oxygen meter. The dissolved oxygen concentration around the periphery of the reactor as well as the time-based change of the corrosion potential of the reactor material have been measured and displayed. Through the hydrogen gas injection from the gas injection system 8 the dissolved oxygen concentration falls, and the corrosion potential also decreases which shows that a sufficient water quality diagnosis and control is effected.

The analysis of dissolved hydrogen, oxygen and hydrogen peroxide in the reactor using the plural kinds of voltammetries carried out in this embodiment, and the following embodiment is performed with the following sequence.

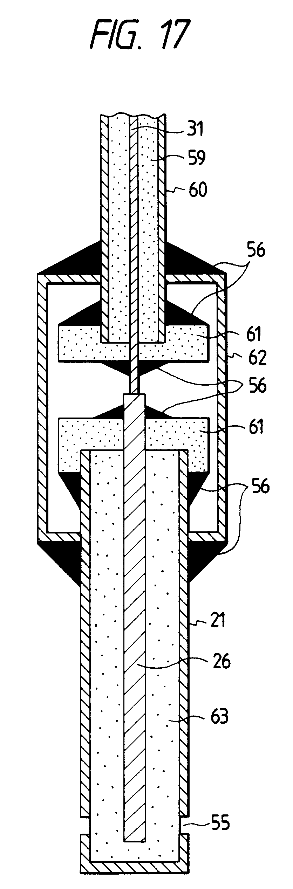

By using normal pulse voltammetry and reverse pulse voltammetry in the present embodiment separation and determination of dissolved hydrogen, dissolved oxygen and dissolved hydrogen peroxide are carried out. In the case where the inner reference electrode (ECP sensor) of the present invention as shown in Fig.2 or in Fig.17 is used as the reference electrode and the counterelectrode for electrochemical measurement, and the platinum disk microelectrode is used as the working electrode, the total diffusion limiting currents in oxidation and reduction of the solution including DH, DO, and $DH_2O_2$ are expressed by the following equations with regard to the respective modes of the normal pulse voltammetry and the reverse pulse voltammetry:

$$I^C\ell_{,NP} = \sum_j issf[P(t_p)]_j, \quad j = H_2O_2, O_2 \quad \ldots(1)$$

$$I^A\ell_{,NP} = -\sum_j issf[P(t_p)]_j, \quad j = H_2O_2, H_2 \quad \ldots(2)$$

$$I^A\ell_{,RP} = -\sum_j issf[P(\tau)]_j, \quad j = H_2O_2, H_2 \quad \ldots(3)$$

$$I^c_{\ell,RP} = iss_{(o_2)}f[P(tp)]_{(o_2)} - iss_{(H_2O_2)}f[P(\tau)]_{(H_2O_2)} + 2iss_{(H_2O_2)}\{f[P(tp)]_{(H_2O_2)} - f[P(\tau)]_{(H_2O_2)}\} \quad (4)$$

Wherein $I^C_{\ell,NP}$ and $I^A_{\ell,NP}$, in the equations (1) and (2) are respectively the total diffusion limiting current for the reduction reaction of $H_2O_2$ and $O_2$, and the total diffusion limiting current for the oxidation reaction of $H_2O_2$ and $H_2$ in the normal pulse voltammetry. $I^A\ell_{,RP}$, and $I^C\ell_{,RP}$ respectively show the total diffusion limiting current for the oxidation reaction of $H_2O_2$ and $H_2$ and the total diffusion limiting current for the reduction reaction of $H_2O_2$ and $O_2$ in the reverse pulse voltammetry.

$$f(P) = \sqrt{\pi}/4P + \pi/4 + 0.094\sqrt{P} \quad (5),$$

wherein $p=4Djtp/r^2$, D is the diffusion coefficient of the respective reaction species j, tp is the pulse width of the normal pulse and the reverse pulse, and r is the radius of the disk microelectrode. $\tau$ in equations (3) and (4) is the retention time of the initially set potential of the reverse pulse voltammetry, in other words, the so-called generation time. Equation (5) is valid up to a radius of the microelectrode of $r=50\mu m$, and tp and $\tau$ are about 600 ms. iss indicates a steady current at the microelectrode and is expressed as $iss = 4nFDjrC°j$, wherein n is the total reaction electron number of the respective oxidation and reduction reaction with regard to oxygen, hydrogen, and hydrogen peroxide, F is the Faraday constant, and $C°j$ is the concentration of the respective chemical species j.

Here, when $I^c\ell_{,NP}$ of equation (1) is subtracted from $I^c\ell_{,RP}$ of equation (2), the following equation is obtained:

$$I^c_{\ell,RP} - I^c_{\ell,NP}$$
$$= \xi(tp,\pi)\cdot 8C°_{(H_2O_2)}FrD_{(H_2O_2)} \quad (6).$$

By indicating $I^c\ell_{,RP} - I^c\ell_{,NP}$ on the ordinate and $\xi(tp,\tau)$ on the abscissa and determining and plotting $\xi(tp,\tau)$ for several pulse widths of the normal pulse and the reverse pulse, $C°_{(H_2O_2)}$, i.e. the hydrogen peroxide concentration, is obtained by calculation from the inclination of the obtained straight line. The diffusion coefficient $D_{(H_2O_2)}$ of hydrogen peroxide is determined in the laboratory beforehand. The following equation applies:

$$\xi(tp, \tau) = \sqrt{\pi r}(1/\sqrt{tp} - 1/\sqrt{\tau})/4\sqrt{D} + 0.188\sqrt{D}(\sqrt{tp} - \sqrt{\tau})/r.$$

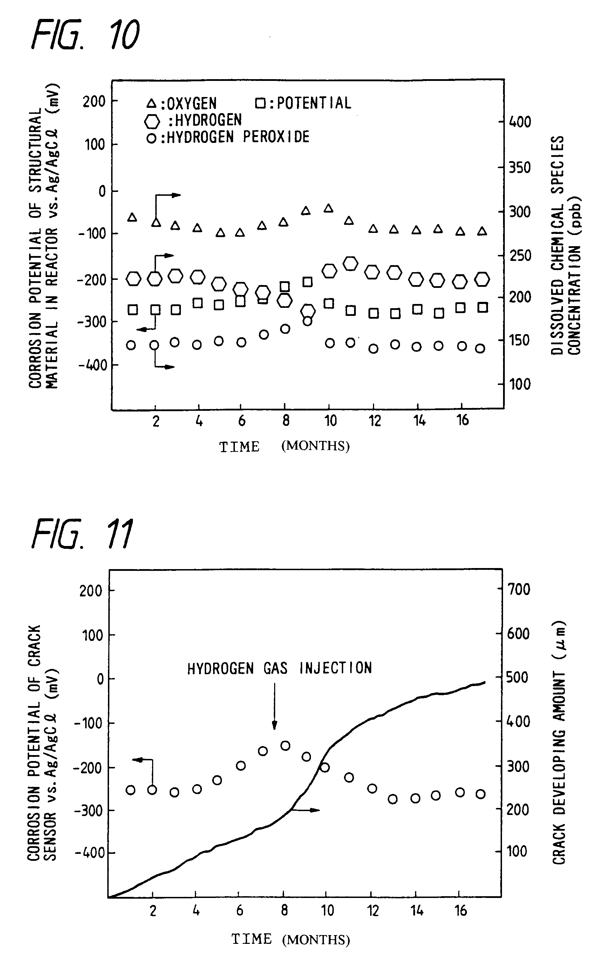

Herein, once hydrogen peroxide is separated and determined with the sequence shown in equation (6), the remaining hydrogen concentration $C°_{H_2}$ is determined by substituting $C°_{(H_2O_2)}$ in equations (2) and (3). Further, when substituting $C°_{(H_2O_2)}$ in equation (1) the oxygen concentration $C°_{O_2}$ is determined. All the above arithmetic processings are programmed in advance in the arithmetic processing automatic control device of the computer 6 and are automatically carried out. The measurement results shown in Fig.8, Fig.9 and Fig.10 are results displayed by using the analysis sequence indicated in the present embodiment.

According to the present invention, direct data relating to the high temperature water in the plant are sampled so that the data reliability is extremely increase, and further direct data of the high temperature water to be monitored are continuously sampled so that the accuracy of the plant operating condition monitoring is enhanced.

Embodiment 5

The present embodiment uses a platinum microelectrode in the reactor instrumentation piping 4 as hydrogen peroxide sensor and measures the hydrogen peroxide concentration in situ in and near the reactor through the electrochemical measurement system as structured in Fig.16 by using the inner reference electrode as shown in Fig.2, Fig.6 and Fig.17. When hydrogen gas is injected from the injection system 8 under the computer control during the slight rise of the hydrogen peroxide concentration as shown in Fig.9, an apparent fall of hydrogen peroxide concentration is observed at the display system and accompanying thereto the corrosion potential tends to decrease slightly. Since this hydrogen peroxide cannot be discriminated from the dissolved oxygen and hydrogen through the usual electrochemical analysis, they were separated and determined by performing plural kinds of pulse voltammetries and arithmetic processing.

Embodiment 6

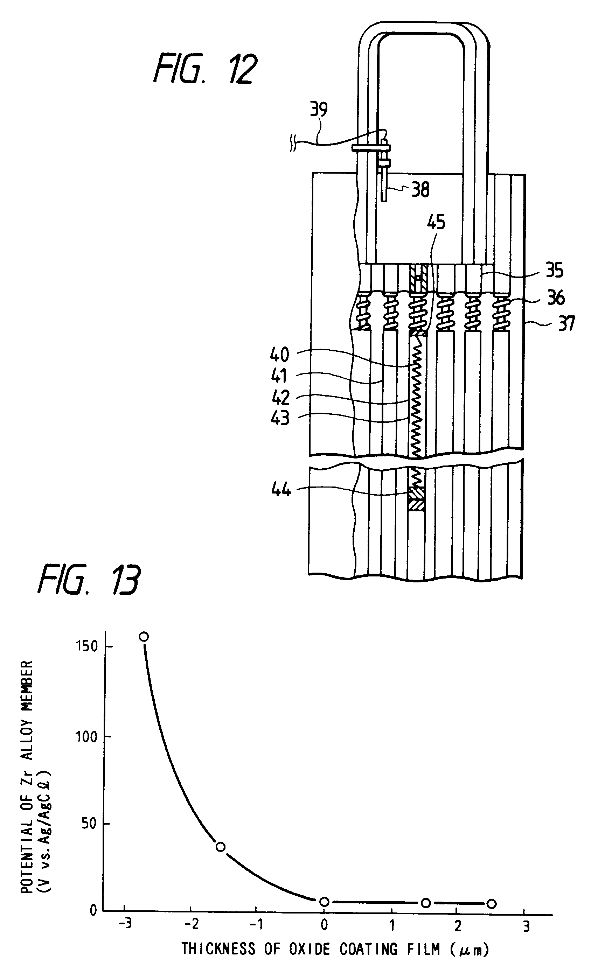

In this embodiment, a crack sensor is installed in the reactor instrumentation piping 4, the inner reference electrode (ECP sensor) as shown in Fig.2 and Fig.6 is jointly used, and the relationship between the crack development amount (M) of the SUS304 steel in the reactor water environment and its corrosion potential is observed at the display system. As shown in Fig.11, the corrosion potential and the crack development amount M due to stress corrosion cracks (SCC) are correlated, and it is understood that the development speed is high in the period when the corrosion potential is high. By injecting hydrogen gas through the gas injection system 8 the crack development amount tends to slightly decrease. Thereby, it is understood that a sufficient water quality diagnosis and control are carried out.

Embodiment 7

In this embodiment, near the fuel assembly as shown in Fig.12 the inner reference electrode (ECP sensor) as shown in Fig.2 is disposed. In Fig.12, uranium pellets 44 are filled into a fuel cladding tube 42 made of Zr alloy and fixed by fuel holding means 40 and an end plug 45 to constitute a fuel element 41. In a channel box 37, many elements are gathered and held by bolts 36 and spacers (not shown) to constitute the fuel assembly. A sensor 38 is, for example, fixed at the upper tie-plate, and the water quality data are taken out through a cable 39.

This embodiment is an example of monitoring the oxide coating thickness of the Zr alloy material by measuring the corrosion potential of the fuel cladding tube made of Zr alloy. Since laboratory data on the relationship between the corrosion potential of the Zr alloy member and the oxide coating thickness are putin in advance, based on its reference value the oxide coating thickness of the cladding tube is immediately determined from the measurement results of the corrosion potential by using the display system 7. The results are illustrated in Fig.13.

Embodiment 8

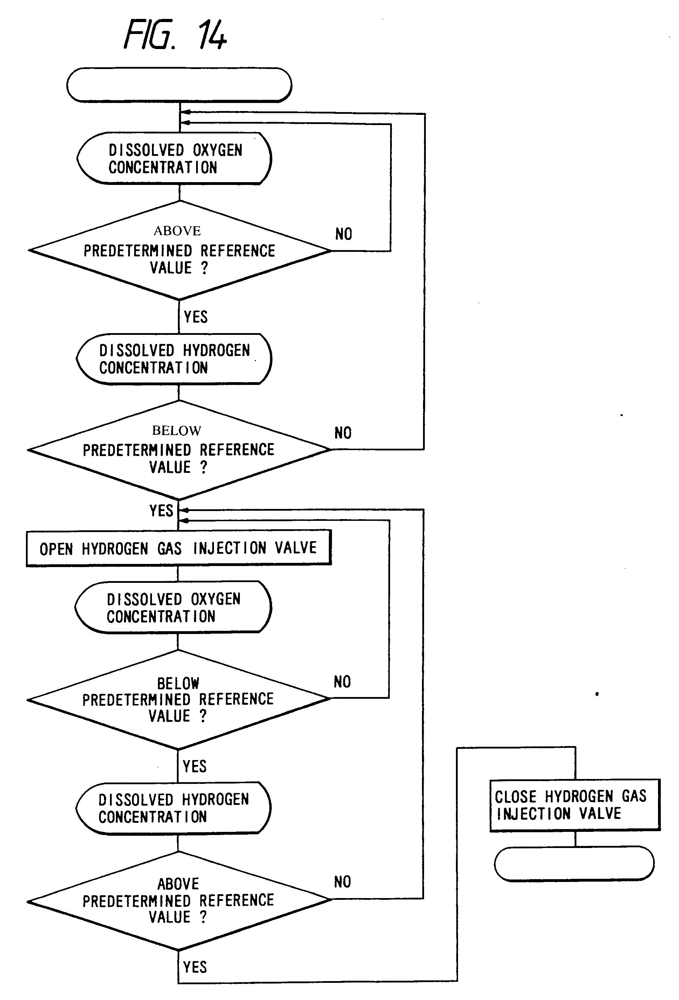

Fig.14 is a flow chart of a continuous water quality control during continuous monitoring of dissolved oxygen and hydrogen in the reactor water by installing the electrochemical measurement system shown in Fig.16 into the instrumentation piping 4 in the reactor. When the dissolved oxygen concentration in the nuclear reactor water exceeds a predetermined reference value, and when the dissolved hydrogen concentration is below a predetermined reference value, the hydrogen gas injection is started through the gas and chemical injection line 8 by opening the hydrogen gas injection valve. Thereafter, the difference amounts of the dissolved oxygen and hydrogen concentrations to the respective reference values are again determined, and the hydrogen gas injection is continued until the conditions are satisfied that the dissolved oxygen concentration be lower and the dissolved hydrogen concentration be higher than the predetermined values.

Embodiment 9

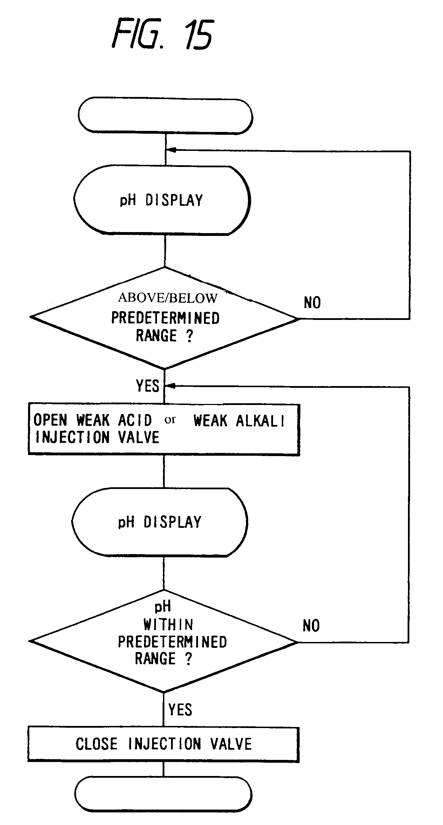

In this embodiment, the electrochemical measurement system shown in Fig.16 is installed in the instrumentation piping 4 of the nuclear reactor shown in Fig.1, where a pH sensor having the structure as shown in Fig.2 is used, wherein a platinum wire instead of a silver wire, 26, and thallic oxide instead of silver halide 23 are used. Fig.15 shows a treatment flow chart for continuously monitoring the pH in the reactor water and maintaining the pH of the reactor water within a predetermined range by injecting weak acid and weak alkali. The weak alkali injection is performed when the pH is below the predetermined range, and the weak acid injection is performed when the pH is above the predetermined range.

11

Embodiment 10

Fig.16 is a cross-sectional view of an electrochemical cell in a neutron instrumentation tube. In this embodiment, a platinum microwire of 20μm in diameter is used as a working electrode. A case 53 encasing respective electrodes of the electrochemical cell is made of SUS316 steel. On the top of the case 53 are communication apertures 55 permitting passage of the cooling water in the reactor and the liquid therein. The apertures facilitate gathering information regarding the cooling water in the reactor and minimize fluctuations in the electrochemical analysis, measurements of pH and corrosion potentials due to convection in the electrochemical cell caused by an inflow of incore water.

The system comprises a low-impedence silver/silver ion reference electrode 2. A working electrode 50 in U form is made of a platinum wire of 20μm in diameter. The system further comprises a platinum/thallic oxide ($Tl_2O_3$) pH electrode 54, the potential of which corresponds to the concentration of hydrogen ions in the reactor water. The counterelectrode 52 is made of a platinum wire of 50μm. A sealing member 52 prevents a decrease in the insulation resistance between the sheath and the core wiring of a mineral insulator (MI) cable 57 due to a leakage of incore water at the time of connecting the core wire and the counterelectrode by metallizing aluminum oxide ($Al_2O_3$) with tungsten. The sealing member 52 of the working electrode 50 has the same function.

The electrode system comprises welded portions 56. As is clear from Fig.16, although the reactor water directly permeates into the upper portion of the cell vessel through the apertures 55, it cannot permeate into the lower space confronting separated by a separation structure.

The four MI cables in the lower space being plugged in the metal tube 58 and led out of the reactor through the instrumentation piping 4, are connected to a computer by means of an ordinary interface technology through a potentiostat and function generator 5 as shown in Fig.1. A variety of electrochemical measurements such as pulse voltammetry, random pulse voltammetry and the like can be performed. Further, by connecting a lead wire with the structure member which is short-circuited to the welded portions 56 and the metal tube 58, and by measuring the difference in the potential between the electrode 2 and the same by using an electrometer, the corrosion potential of the stainless steel under the environment of the water quality of a reactor can be measured.

Further, by connecting the electrometer between the reference electrode 2 and the pH electrode 54, the potential can be measured. Thereby, by measuring the difference in the potential between the reference electrode 2 and the pH electrode 54, the pH of the reactor water is obtained. In order to satisfy the requirement for compactness so as to be installed in a neutron instrumentation, and to reduce impedance due to the resistance of the cell and the like during measurement in high temperature and high pressure water with low electrical conductivity, the four electrodes 2, 50 ,54 and 51 are disposed in close proximity. The inner cell diameter of the vessel 53 filled with reactor water is 20 mm. The four electrodes are disposed in a circular arrangement in the cell as close as possible.

Fig.17 is an enlarged detail view of the reference electrode 2 shown in Fig.16. This electrode is composed of a silver/silver ion electrode. The porous aluminum oxide layer 63 surrounding the silver wire 26 is composed of compression moulded powdered aluminum oxide (300 mesh pass). The porous layer 63 is filled with reactor water passing through an aperture 55 of about 0.5 mm in diameter, and stores silver ions dissolving from the silver wire. The diffusion speed of silver ions diffusing from the compression moulded layer 63 into the reactor water through the liquid communication aperture 55 varies depending on the compression pressure applied on the mould. By adjusting the compression pressure, the diffusion speed has been controlled to be below $10^{-12}$ mol/cm²·s.

The silver/silver ions electrode comprises a sheath 21. A seal 61 made of tungsten-metallized alumina prevents a decrease in the insulation resistance due to a leakage of reactor water. The arrangement further comprises an alumina insulation layer 59. An MI cable 31 of SUS304 serves as a lead wire for the silver wire 26. The sheath CO of the MI cable is made of the same SUS304 steel as the cable. An adapter 62 serves for connecting the MI cable with the electrode member.

Embodiment 11

In this example embodying the present invention, an electrochemical sensor as shown in Fig.16 is used, which is installed in place of the ECP sensor in Fig.1, and by applying random pulse voltammetry and a neural network, the concentrations of dissolved oxygen, hydrogen peroxide and hydrogen ($C°_{(O_2)}$, $C°_{(H_2O_2)}$, $C°_{(H_2)}$) are analysed quantitatively and simultaneously.

The random pulse voltammetry is a voltammetry to which the pattern recognition method is applied. In the voltammetry shown applied in embodiment 4 wherein a normal pulse and a reverse pulse are used in combination, the measurement of the concentration of each chemical species depends on variable one-dimensional information on a diffusion limiting current. In this embodiment, however, by fluctuating a system to measure in multi-modes with more complicated fluctuation signals (random pulse potential signals), an attempt to obtain as much of electrolytic current data (pattern information) as possible including information on electrochemical reactions of each system to measure has been made. Namely, even with such chemical species as the above wherein the response potentials between them are very close and difficult to separate for measurement, by focusing on their characteristic patterns or characteristic vectors obtained, by means of a data compression processing explained below, based on the differences in the speed of their charge transfer, parameters and the like among other electrochemical reaction processes, their quantitative and qualitative analyses are obtained, the former from the size and the measured line of a characteristic vector, the latter from the difference in the appearance patterns of a characteristic vector.

The random pulse voltammetry is executed in order of (1) gathering of pattern information (electrolysis current data), (2) compression of pattern data, (3) extraction of object information from compressed pattern information $C^{\circ}_{(O_2)}$, $C^{\circ}_{(H_2O_2)}$, $C^{\circ}_{(H_2)}$ values. The outline of each of steps of (1), (2) and (3) is as follows. In step (1), electrolysis currents of oxygen, hydrogen peroxide and hydrogen are measured as response signals corresponding to a random pulse potential applied to a measuring system. In this embodiment, a potential signal of a random pulse is expressed by using two potential waveform parameters (m,n), namely, a potential corresponding to the pulse potential of the first random pulse represented by m, and another one corresponding to the pulse potential of the second random pulse expressed by n. Therefore, by taking m on the x axis, and n on the y axis, the electrolysis currents, i.e., response signals responding to each input potential on the x,y coordinates, are measured and arranged in form of a matrix corresponding to the values of the parameters. In step (2), a data matrix consisting of 64 (8x8) points representing the current component of a response signal obtained in step (1) corresponding to an input potential signal is transformed and compressed into data vectors with several components. As means for data compression, there are Hadamard transformation, Karhunen-Loéve transformation, Haar transformation and the like used in Fourier transformation or in image processing. In this embodiment, the two-dimensional Hadamard transformation is used. In step (3), by extracting characteristic vectors from the data vectors obtained in step (2), $C^{\circ}_{(O_2)}$, $C^{\circ}_{(H_2O_2)}$, $C^{\circ}_{(H_2)}$ are determined. That is, through entering each component from the obtained data vectors, functions capable of outputting corresponding $C^{\circ}_{(O_2)}$, $C^{\circ}_{(H_2O_2)}$, $C^{\circ}_{(H_2)}$ are sought, and set up. In this embodiment, the function is determined by using a neural network. Each step of (1), (2) and (3) in this embodiment is explained below in detail.

First, "gathering of pattern information" in step (1) is explained in detail.

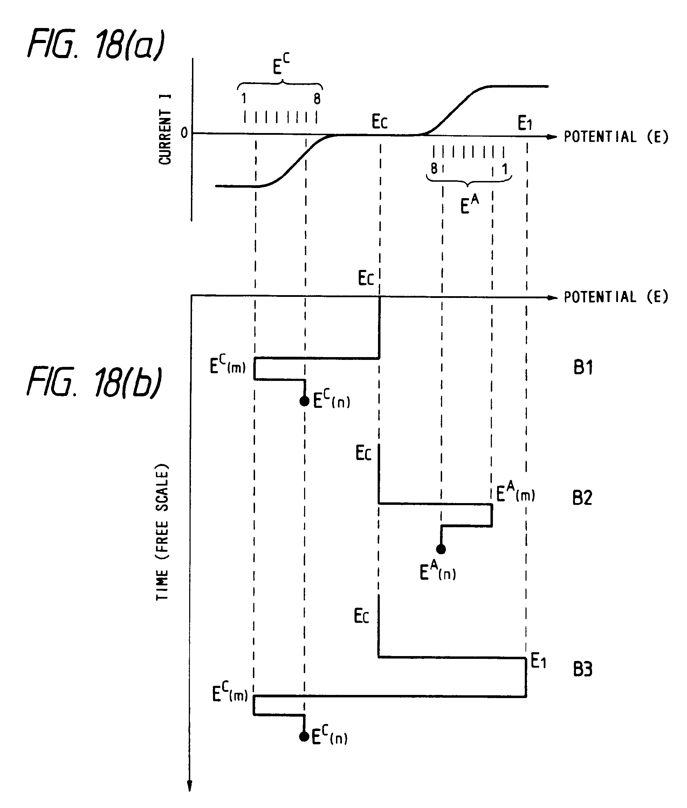

Fig.18 explains a method for applying a random pulse potential signal. Nevertheless, simply applying random pulse potential signals sometimes fails to give information on $C^{\circ}_{(O_2)}$, $C^{\circ}_{(H_2O_2)}$, $C^{\circ}_{(H_2)}$. Considering that the voltammetry as described in embodiment 4 using a normal pulse and a reverse pulse in combination allows to separate and determine $C^{\circ}_{(O_2)}$, $C^{\circ}_{(H_2O_2)}$, $C^{\circ}_{(H_2)}$, a random pulse potential signal method based on the above combined pulse application method has been devised in this embodiment. Fig.18 is a schematic diagram showing a current (i)-potential (E) curve in a three species mixture system of oxygen, hydrogen peroxide and hydrogen. In the positive potential region, oxidizing currents of hydrogen peroxide and hydrogen are observed, while in the negative region, reducing currents of oxygen and hydrogen peroxide are observed, respectively. Potential regions wherein oxidizing currents and reducing currents are observed are defined as $E^A$ and $E^C$, respectively, and divided into the number $2^{\ell}$ ($\ell$=1, 2, 3,...). The adoption of the number $2^{\ell}$ is for ease of processing by a computer. In this embodiment, they are divided into 8 levels ($\ell$=3) respectively, and each level is expressed by $E^A(n)$ and $E^C(n)$ (n=1, 2, 3...8). Further, a potential at which no electrode reaction of oxygen, hydrogen peroxide or hydrogen occurs is defined as $E_C$, and a potential at which a diffusion limiting current of an oxidation reaction of hydrogen peroxide and hydrogen is obtained is defined as $E_1$.

B1 ~ B3 in Fig.18 (b) are three kinds of random pulse potential signals to be applied to the working electrode in the electrochemical sensor. These random pulse signals of three kinds are generated based on a normal pulse for obtaining $I^C_{\ell,NP}$ in embodiment 4, a normal pulse for obtaining $I^A_{\ell,NP}$, and a reverse pulse for obtaining $I^C_{\ell,RP}$.

The first kind of random pulse is applied in the order of $E_c \rightarrow E^c (m) \rightarrow E^c (n)$. In this embodiment $E^c$ is applied for one second, and $E^c (m)$ and $E^c (n)$ are applied for 0.05 second, respectively, and sampling of current values is conducted on completing $E^c (n)$. m and n each represent a level (1 ~ 8) of division of $E^c$. There are 64 (8x8) combination patterns for the potential of $E^c (m)$ and $E^c (n)$. Each current value corresponding to a combination of m and n is stored in an 8x8 data matrix $X^c_{NP}$. Because the thus obtained $X^c_{NP}$ matrix comprises operational data on the reduction reactions of oxygen and hydrogen peroxide in the measured water, it provides almost the same quality of information on $C^{\circ}_{(O_2)}$, $C^{\circ}_{(H_2O_2)}$ as provided by $I^c\ell,_{NP}$ in embodiment 4. $X^c_{NP}$, however,

provides a larger quantity of information than $I^c\ell_{,N}p$, because the former includes existing data of as many as 64 points.

The second kind of random pulse is applied in the order of $E_c \rightarrow E^A(m) \rightarrow E^A(n)$. In this embodiment $E_c$ is applied for one second, and $E^A(m)$ and $E^A(n)$ are applied for 0.05 second, respectively, and the sampling of the current values is conducted on completing $E^A(n)$. In this case like the first one, an 8x8 data matrix $X^A_Np$ corresponding to each combination of m and n is obtained. Because the thus obtained $X^A_Np$ matrix comprises existing data on the oxidation reactions of hydrogen peroxide and hydrogen in the measured water, it provides almost the same quality of information on $C^{\circ}_{(H_2O_2)}$, $C^{\circ}_{(H_2)}$, as provided by $I^A\ell_{,N}p$ in embodiment 4.

The third kind of random pulse is applied in the order of $E_c \rightarrow E_1 \rightarrow E^c(m) \rightarrow E^c(n)$. In this embodiment, for the same respective periods of time as for the first kind random pulse, Ec, $E^c(m)$, $E^c(n)$ are applied, and $E_1$ for 0.1 second. After completing $E^c(n)$, the sampling of the current values was conducted. In this case alike for the first and the second kinds, an 8x8 data matrix $X^c_Rp$ corresponding to combinations of m and n is obtained. The third kind random pulse differs from the first kind one in that $E_1$ is applied in each preceding stage of $E^c(m)$ and $E^c(n)$, one after another. This means that $X^c_Rp$ includes information on the reduction reaction of oxygen generated through the oxidation of hydrogen peroxide in $E_1$, in addition to the information on the reduction of oxygen and hydrogen peroxide in the tested water, of alike $I^c\ell_{,R}p$ in the embodiment 4.

In the following, "Compression of pattern data" in (2) is explained in detail.

Here the matrix $X^c_{(Rp-Np)}$ is defined by equation (1), before the data matrix is transformed and compressed into

$$X^c_{(Rp-Np)} = X^c_Rp - X^c_Np \quad (1).$$

Through this operation, information on the reduction reactions by oxygen and hydrogen peroxide in the tested water is almost cancelled, whereby the information on the reduction reaction of the oxygen generated by oxidation of hydrogen peroxide is condensed in $X^c_{(Rp-Np)}$. That is, $X^c_{(Rp-Np)}$ provides almost the same quality of information as $I^c\ell_{,R}p - I^c\ell_{,R}p$ in embodiment 4. The data matrices $X^c_{(Rp-Np)}$, $X^c_Np$, and $X^A_Np$ are transformed into matrices $Y^c_{(Rp-Np)}$, $Y^c_Np$, $Y^A_Np$, respectively by equation (2):

$$Y = H \cdot X \cdot H \quad (2)$$

where,

$$H = \frac{1}{\sqrt{8}} \begin{pmatrix} 1 & 1 & 1 & 1 & 1 & 1 & 1 & 1 \\ 1 & -1 & 1 & -1 & 1 & -1 & 1 & -1 \\ 1 & 1 & -1 & -1 & 1 & 1 & -1 & -1 \\ 1 & -1 & -1 & 1 & 1 & -1 & -1 & 1 \\ 1 & 1 & 1 & 1 & -1 & -1 & -1 & -1 \\ 1 & -1 & 1 & -1 & -1 & 1 & -1 & 1 \\ 1 & 1 & -1 & -1 & -1 & -1 & 1 & 1 \\ 1 & -1 & -1 & 1 & -1 & 1 & 1 & -1 \end{pmatrix} \quad \cdots (3)$$

assuming that each component of the matrix Y is expressed by

$$Y = \begin{pmatrix} y_{11} & y_{12} & \cdots & y_{1\bullet} \\ y_{21} & y_{22} & \cdots & y_{2\bullet} \\ \vdots & \vdots & & \vdots \\ y_{\bullet 1} & y_{\bullet 2} & \cdots & y_{\bullet\bullet} \end{pmatrix} \quad \cdots (4) \quad .$$

Information belonging to X appears on a specific portion of Y consisting of 64 components, i.e., information on X dispersed on the 64 components of the same, is compressed into a specific set of several numbers of components in Y. More specifically, in this embodiment, information on X is compressed on Y components, $y_{11}, y_{15}, y_{51}, y_{17}, y_{71}$. Further, from the components, $y_{11}, y_{15}, y_{51}, y_{17}, y_{71}$ for each $Y^c_{(Rp-Np)}$, $Y^c_Np$, $Y^A_Np$ vectors $Z^c_{(Rp-Np)}$, $Z^c_Np$, $Z^A_Np$ are obtained as follows:

$$Z = (y_{11}, y_{15}, y_{51}, y_{17}, y_{71}) \quad (5).$$

It has been thus explained that vectors $Z^c_{(Rp-Np)}$, $Z^c_Np$, and $Z^A_Np$ contain highly compressed information on

$C^\circ_{(H_2O_2)}$, $C^\circ_{(O_2)} + C^\circ_{(H_2O_2)}$, $C^\circ_{(H_2O_2)} + C^\circ_{(H_2)}$, respectively.

Next, "Extraction of object information from compressed pattern information" in (3) is explained in detail in the following.

A scalar field f is provided, to satisfy the following equation 6 in relation to a vector $Z^c_{(RP-NP)}$ obtained for a test water having a discretionary $C^\circ_{(O_2)}$, $C^\circ_{(H_2O_2)}$, $C^\circ_{(H_2)}$ :

$$C^\circ_{(H_2O_2)} = f(Z^c_{(RP-NP)}) \quad (6).$$

Scalar fields f are obtained beforehand for several kinds of test water having a discretionary $C^\circ_{(O_2)}$ and $C^\circ_{(H_2)}$ for known $C^\circ_{(H_2O_2)}$. In this embodiment, this scalar field f is determined by means of a neural network system wherein each component of the vector $Z^c_{(RP-NP)}$ is entered as input, and $C^\circ_{(H_2O_2)}$ is put out. Even for a test water with an unknown value of $C^\circ_{(H_2O_2)}$, this value can be calculated by equation 6.

In the same manner, scalar fields g and h are provided to satisfy the following equations (7) and (8) in relation to the vectors $Z^c_{NP}$ and $Z^A_{NP}$ obtained for a test water having a discretionary $C^\circ_{(O_2)}$, $C^\circ_{(H_2O_2)}$, $C^\circ_{(H_2)}$ :

$$C^\circ_{(O_2)} = g(Z^c_{NP}) - C^\circ_{(H_2O_2)} \quad (7)$$

$$C^\circ_{(H_2)} = h(Z^A_{NP}) - C^\circ_{(H_2O_2)} \quad (8).$$

The scalar fields g and h are obtained beforehand for a test water wherein $C^\circ_{(H_2O_2)}$ and $C^\circ_{(O_2)}$ are known, or $C^\circ_{(H_2O_2)}$ and $C^\circ_{(H_2)}$ are known, respectively. In this embodiment, by using a neural network wherein each component of vector $Z^c_{NP}$ is entered as input and $C^\circ_{(O_2)} + C^\circ_{(H_2O_2)}$ is put out, and also another neural network wherein each component of vector $Z^A_{NP}$ is entered and $C^\circ_{(H_2)} + C^\circ_{(H_2O_2)}$ is put out, the scalar fields g and h have been determined. Also for a test water with unknown $C^\circ_{(O_2)}$, $C^\circ_{(H_2O_2)}$ and $C^\circ_{(H_2)}$ these unknown values can be obtained by equation (6), (7) and (8), respectively.

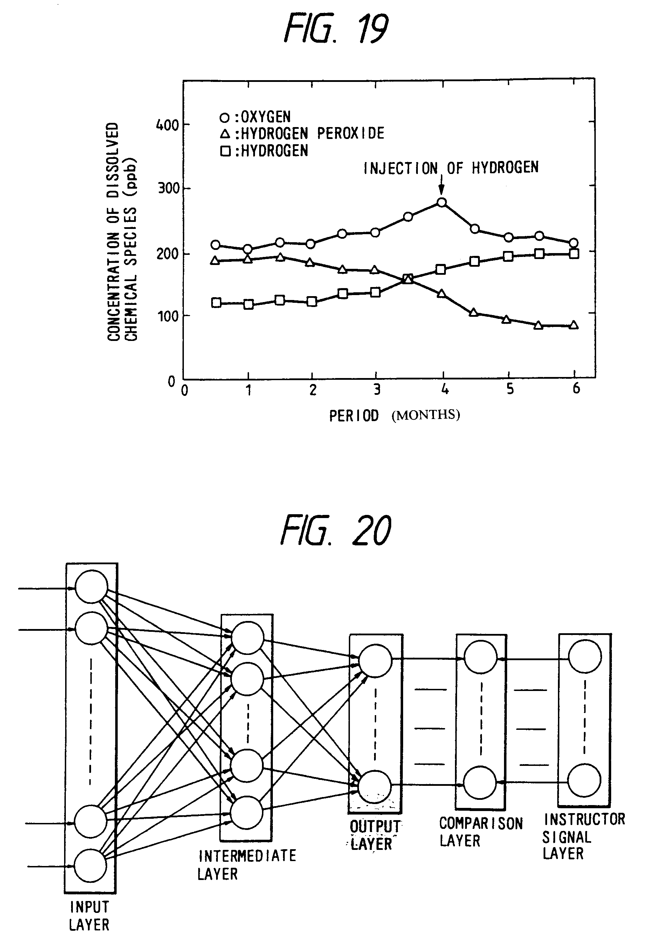

Fig.19 is a schematic diagram illustrating the concentration variations of dissolved oxygen, hydrogen peroxide and hydrogen in the incore water with time, wherein the amounts of these items was concurrently measured my means of the methods described above. When the concentrations of dissolved oxygen and hydrogen peroxide increase, the computer 6 instructs the gas injection system 8 to inject hydrogen according to the sequence shown in Fig.1. It is shown in this figure that hydrogen injection was effective in the reduction of the concentrations of oxygen and hydrogen peroxide.

In the following, a fundamental calculation method for the neural network is described.

First, each of the determined signal values $Z_1 \sim Z_n$ is multiplied by a weighting factor $W_{ji}$, and summed up in the following equation in form of a product·sum:

$$C_{j(z)} = \sum_{i=1}^{n} W_{ji}(2 \leftarrow 1) \cdot Z_i(1) \qquad \ldots (1) ,$$

wherein are: $Z_i(1)$: the value of the input layer (1st layer); $W_{ji}(2 \leftarrow 1)$: a weighting factor from a variable at i number of the input layer (1st layer) to a neuron element model at j number of the intermediate layer (2nd layer); $C_j(2)$: the total value of input at j number neuron element model in the intermediate layer (2nd layer).

For a neuron element model in the input layer, its output value is calculated by the following equation in accordance with a compensation for $C_j(2)$:

$$Z_j(2) = 1/(I - e^{-C_j(2)}) \quad (2).$$

Equation (2) is expressed by a relationship as shown in Fig.20, i.e., a value ranging from "0" to "1" is obtained for $Z_j(2)$ corresponding to the value of $C_j(2)$. The calculated value $Z_j(2)$ is sent to the output layer, wherein the like calculation is executed.

An outline of the calculation in the neural network is given below. The input value $Z_i(1)$ described above is entered in the input layer in Fig.20, and its signal value is put out to a neuron element model in the intermediate layer. In the neuron element model of the intermediate layer, $C_j(2)$ of the product·sum of the same output value $Z_i(1)$, and the weighting factor $W_{ij}(2 \leftarrow 1)$ is calculated by equation (1), and in accordance with its compensation, an output value $Z_j(2)$ to the output layer is determined by equation (2). Likewise, the output value $Z_j(2)$ further serves for calculating $C_j(3)$ as a product·sum with a weighting factor $W_{ij}(3 \leftarrow 2)$ for the intermediate layer (the 2nd layer) and the output layer (the 3rd layer by the following equation:

$$C_j(3) = \sum_{i=1}^{n} W_{ji}(3 \leftarrow 2) \cdot Z_i(2) \qquad \ldots (3) ,$$

wherein is are: $Z_i(2)$: the value at the intermediate layer (the 2nd layer); $W_{ij}(3{\leftarrow}2)$: a weighting factor from a variable at i number in the intermediate layer (the 2nd layer) to a neuron model at j number in the output layer (the 3rd layer); $C_j(3)$: the total sum of input to a neuron element model at j number in the output layer (the 3rd layer).

Further, according to the value of $C_j(3)$, the output value $Z_j(3)$ at the output layer is calculated by the following equation:

$$Z_j(3) = 1/(I - e^{- C_j(3)}) \quad (4).$$

Thereby, a calculated value of $Z_j(3)$ for the output layer is obtained.

In order to execute a learning function in the neural network, a comparison layer and an instructor signal layer as shown in Fig.20 are provided, and a signal from the output layer and a corresponding signal from the instructor signal layer are putin in the comparison layer for comparison. Thereby the values of the weighting factors $W_{ji}(3{\leftarrow}2)$ and $W_{ji}(2{\leftarrow}1)$ are adjusted so as to reduce the difference or error in comparison.

If the calculated signals obtained by equations (1) or (4) and the instructor signals are again compared, there still arise like differences. Thereby once again, weighting factors $W_{ji}(3{\leftarrow}2)$ and $W_{ji}(2{\leftarrow}1)$ are adjusted still further to reduce the differences in the values.

This process of adjustment of weighting factors $W_{ji}$ is repeated until the difference or error becomes sufficiently small. Because these weighting factors are given randomly at first by random number generation, the errors are great in the initial stages. The output signals, however, gradually approach the instructor's signal values. The scalar fields f, g, and h are determined through these procedures. Here, the symbols c and z correspond to a concentration and a characteristic vector, respectively.

Such a method for adjusting errors as described above is called the error reverse propagation method based on the technique devised by Rumelhart. A detailed description of the method is given in "Parallel Distributed Processing" by Rumelhart, MIT Press, vol.1 (1986).

In accordance with the present invention, an electrochemical analysis in a nuclear reactor can be readily performed by means of a low impedance reference electrode. The reference electrode has a structure protected by a microscopically porous layer, wherein the dissolution of reference electrode ions from the reference electrode and of incore water can be controlled to be less than $10^{-12}$mol/cm$^2$·s in volume of flux, providing an extremely long life (over 15 years life time of silver in a 0.5g silver/silver ion reference electrode) and a stable electrochemical analysis. Thereby, advanced technologies existing in the field of electrochemical analysis have been effectively applied to the analysis of the incore water quality, providing in-situ analysis of incore water for the first time as well as adequate incore information. By means of this technology, a plant operation status monitoring system using in-situ incore water quality data has been provided.

Further, due to the development of a water quality sensing technique comprising an electrochemical cell structure of a long life exceeding the regular inspection periods of nuclear plants, and also due to the application of the neural network technique in processing the water quality data, the reliability of the data is substantially increased, and the reliability in the operation status supervisory system has been substantially enhanced.

The electrochemical electrode according to the present invention, unlike the electrochemical cell disclosed in JP-A- 59-177474, does not necessitate the use of a resin membrane of high durability and selective permeability, thus enhancing its use in an environment of high temperatures and high pressure for a long period of time.

Further, in the prior art disclosed in JP-A- 56-77751, by using zirconium oxide and the like as a hydrogen ion sensing element film, the pH of high temperature water is measured. However, because zirconium oxide and the like have an extremely large impedance, they are not preferable in practice. On the other hand, in the present invention, because the ionic electrode member is surrounded by a porous ceramic or porous heat resistant plastic sheath, an electrochemical cell structure having an adequate impedance can be made, without imposing excessive burden on output signal processing circuits and the like.

Because the impedance of the reference and the like electrodes according to the present invention can be made small enough, thus reducing the impedance in a feedback circuit of a control amplifier, an oscillation of output signals due to phase shift is easily prevented.

## Claims

1. A monitoring system for monitoring chemical properties of high temperature water in power plants, comprising
   - an electrochemical sensor (2) being in electrochemical contact with the water for detecting the respective water property,

- means (6) receiving the information sampled from the sensor (2) for analyzing the respective water property on the basis of the sampled information and for comparing the obtained results with predetermined reference values for the plant operation and manipulation, and
- means (7) for displaying and/or recording necessary portions of the comparison results, **characterized** in that
  - the electrochemical sensor (2) is disposed in direct contact with the high temperature water, and
  - the electrochemical sensor (2) is in electrochemical contact with the water through a porous material (22) which substantially suppresses dissolution of the sensor electrolyte (23) into the high temperature water, but is ion-permeable (Fig. 1, 2).

2. The monitoring system according to claim 1,
   characterized in that a further porous material (24) is provided on the porous material (22).

3. The monitoring system according to claim 1 and/or 2,
   characterized in that the electrochemical sensor (2) is a reference electrode (ECP), a pH electrode (pH), an electrochemical sensor for detecting dissolved hydrogen (DH), dissolved oxygen (DO) or dissolved hydrogen peroxide ($DH_2O_2$), a sensor for detecting the conductivity of the water (S), or a crack sensor (M).

4. The monitoring system according to claim 3,
   characterized in that the porous material (22, 24) is a ceramic or a resin material.

5. The monitoring system according to one or several of claims 1 to 4,
   characterized in that the electrochemical sensor (2) is disposed in a recirculation water line (11), a storage water line, an instrumentation piping (4) of a reactor core (3), the reactor core (3) or its vicinity, a separator or its vicinity, a mixing plenum chamber or its vicinity, a downcomer or its vicinity, a jet pump or its vicinity, a downstream plenum chamber or its vicinity, or a cooling water piping line.

6. The monitoring system according to one or several of claims 1 to 5,
   characterized in that it comprises means (6) for predicting a variation of the reference value based on the mutual relationship between the reference value for the plant operation and manipulation and the data sampled from the sensor (2), and a gas and chemical injection system (8) for adjusting the water quality.

7. The monitoring system according to claim 6,
   characterized in that means (7) are provided for selecting a predetermined reference value for the plant operation and manipulation in response to the determined water quality.

8. Electrochemical electrode for detecting electrochemical properties of solutions, comprising
   - an electrode member (26) which is in electrochemical contact with an electrolyte (23),
   - a terminal (31) for electrically contacting the electrode member (26), and
   - a non-liquid-permeable porous ceramic or resin material (22) disposed on the electrolyte (23),
   characterized in that a further porous material (24) is provided on the porous material (22) (Fig. 2).

9. The electrode according to claim 8,
   characterized in that it is a reference electrode, the electrolyte (23) is a solid and contains ions of the electrode member (26), the porous material (22) is a ceramic or resin material, and the further porous material (24) is a heat resistant ceramic material.

10. The electrode according to claim 8 and/or 9,
    characterized in that a sheath (21) is provided on the further porous material (24) which has apertures allowing contact of the further porous material (24) with the solution to be measured.

**Patentansprüche**

1. Überwachungssystem zur Überwachung chemischer Eigenschaften von Hochtemperaturwasser in Kraftwerken, mit

- einem elektrochemischen Sensor (2), der in elektrochemischem Kontakt mit dem Wasser steht, um die jeweilige Eigenschaft des Wassers zu erfassen,
- Mitteln (6), die die vom Sensor (2) abgenommene Information empfangen, um die jeweilige Eigenschaft des Wassers anhand der abgenommenen Information zu analysieren und die erhaltenen Ergebnisse mit vorgegebenen Referenzwerten für Betrieb und Führung des Kraftwerks zu vergleichen, und
- Mitteln (7) zum Anzeigen und/oder Aufzeichnen von benötigten Teilen der Vergleichsergebnisse,

dadurch gekennzeichnet, daß

- der elektrochemische Sensor (2) in direktem Kontakt mit dem Hochtemperaturwasser ist und
- der elektrochemische Sensor (2) mit dem Wasser durch ein poröses Material (22) in Kontakt ist, das das Auflösen des Sensorelektrolyten (23) im Hochtemperaturwasser im wesentlichen unterdrückt, das aber ionendurchlässig ist (Fig. 1, 2).

2. Überwachungssystem nach Anspruch 1, dadurch gekennzeichnet, daß ein weiteres poröses Material (24) an dem porösen Material (22) vorgesehen ist.

3. Überwachungssystem nach Anspruch 1 und/oder 2, dadurch gekennzeichnet, daß der elektrochemische Sensor (2) eine Referenzelektrode (ECP), eine pH-Elektrode (pH), ein elektrochemischer Sensor zum Erfassen von gelöstem Wasserstoff (DH), gelöstem Sauerstoff (DO) oder gelöstem Wasserstoffperoxid ($DH_2O_2$), ein Sensor zum Erfassen der Leitfähigkeit des Wassers (S) oder ein Rißsensor (M) ist.

4. Überwachungssystem nach Anspruch 3, dadurch gekennzeichnet, daß das poröse Material (22, 24) ein Keramik- oder Harzmaterial ist.

5. Überwachungssystem nach einem oder mehreren der Ansprüche 1 bis 4, dadurch gekennzeichnet, daß der elektrochemische Sensor (2) in einer Wasserkreislaufleitung (11), einer Speicherwasserleitung, einer Meß- oder Regelleitung (4) eines Reaktorkerns (3), dem Reaktorkern (3) oder seiner Umgebung, einem Wasserabscheider oder seiner Umgebung, einer Verteilerkammer oder ihrer Umgebung, einer Rücklaufleitung oder ihrer Umgebung, einer Strahlpumpe oder ihrer Umgebung, einer Sammelkammer oder ihrer Umgebung oder einer Kühlwasserrohrleitung angebracht ist.

6. Überwachungssystem nach einem oder mehreren der Ansprüche 1 bis 5, dadurch gekennzeichnet, daß es Mittel (6) zum Vorhersagen einer Schwankung des Referenzwerts auf Grundlage der Beziehung zwischen dem Referenzwert für den Betrieb und die Führung des Kraftwerks und den vom Sensor (2) abgenommenen Daten sowie ein Gas- und Chemikalien-Einspritzsystem (8) zum Einstellen der Wasserqualität umfaßt.

7. Überwachungssystem nach Anspruch 6, dadurch gekennzeichnet, daß Mittel (7) vorgesehen sind zum Auswählen eines vorgegebenen Referenzwertes für Betrieb und Führung des Kraftwerks in Abhängigkeit von der ermittelten Wasserqualität.

8. Elektrochemische Elektrode zum Bestimmen elektrochemischer Eigenschaften von Lösungen, mit
- einem Elektrodenelement (26), das in elektrochemischem Kontakt mit einem Elektrolyten (23) ist,
- einem Anschluß (31) zum elektrischen Kontaktieren des Elektrodenelements (26), und
- einem flüssigkeitsundurchlässigen porösen Keramik- oder Harzmaterial (22), das an dem Elektrolyten (23) angebracht ist,

dadurch gekennzeichnet, daß ein weiteres poröses Material (24) an dem porösen Material (22) vorgesehen ist (Fig. 2).

9. Elektrode nach Anspruch 8, dadurch gekennzeichnet, daß sie eine Referenzelektrode ist, der Elektrolyt (23) ein Festkörper ist und Ionen des Elektrodenelements (26) enthält, das poröse Material ein Keramik- oder Harzmaterial ist und das poröse Material (24) ein hitzefestes Keramikmaterial ist.

10. Elektrode nach Anspruch 8 oder 9, dadurch gekennzeichnet, daß eine Scheide (21) an dem weiteren porösen Material (24) vorgesehen ist, die Öffnungen besitzt, die den Kontakt des weiteren porösen Materials (24) mit der zu messenden Lösung ermöglichen.

## Revendications

1. Système de contrôle pour contrôler des propriétés chimiques d'une eau à haute température dans des centrales électriques, comprenant
   - un capteur électrochimique (2) en contact électrochimique avec l'eau pour détecter la propriété respective de l'eau,
   - des moyens (6) recevant l'information échantillonnée à partir du capteur (2) pour analyser la propriété respective de l'eau sur la base de l'information échantillonnée et pour comparer les résultats obtenus à des valeurs de référence prédéterminées pour le fonctionnement et la manoeuvre de la centrale, et
   - des moyens (7) pour afficher et/ou enregistrer des parties nécessaires des résultats de comparaison,

   caractérisé en ce que
   - le capteur électrochimique (2) est placé en contact direct avec l'eau à haute température, et
   - le capteur électrochimique (2) est en contact électrochimique avec l'eau par l'intermédiaire d'un matériau poreux (22) qui supprime pour l'essentiel la dissolution de l'électrolyte (23) du capteur dans l'eau à haute température, mais est perméable aux ions. (Figures 1, 2).

2. Système de contrôle selon la revendication 1, caractérisé en ce qu'un autre matériau poreux (24) est prévu sur le matériau poreux (22).

3. Système de contrôle selon la revendication 1 et/ou 2, caractérisé en ce que le capteur électrochimique (2) est une électrode de référence (ECP), une électrode (pH) de mesure du pH, un capteur électrochimique pour détecter l'hydrogène dissous (DH), l'oxygène dissous (DO) ou le peroxyde d'hydrogène dissous ($DH_2O_2$), un capteur pour détecter la conductivité de l'eau (S) ou un détecteur de fissures (M).

4. Système de contrôle selon la revendication 3, caractérisé en ce que le matériau (22,24) est une céramique ou un matériau formé d'une résine.

5. Système de contrôle selon une ou plusieurs des revendications 1 à 4, caractérisé en ce que le capteur électrochimique (2) est disposé dans une canalisation d'eau de recirculation (11), une canalisation d'eau de stockage, une canalisation d'instrumentation (4) d'un coeur de réacteur (3), le coeur de réacteur (3) ou son voisinage, un séparateur ou son voisinage, une chambre de collecte de mélange ou son voisinage, une canalisation descendante ou son voisinage, une pompe à jet ou son voisinage, une chambre de collecte aval ou son voisinage ou une canalisation d'eau de refroidissement.

6. Système de contrôle selon une ou plusieurs des revendications 1 à 5, caractérisé en ce qu'il comprend des moyens (6) pour prédire une variation de la valeur de référence sur la base de la relation mutuelle entre la valeur de référence pour le fonctionnement et la manoeuvre de la centrale et les données échantillonnées provenant du capteur (2), et un système (8) d'injection de gaz et d'un produit chimique pour ajuster la qualité de l'eau.

7. Système de contrôle selon la revendication 6, caractérisé en ce que des moyens (7) sont prévus pour sélectionner une valeur de référence prédéterminée pour le fonctionnement et la manoeuvre de la centrale en réponse à la qualité déterminée de l'eau.

8. Électrode électrochimique pour déterminer des propriétés électrochimiques de solutions, comprenant :
   - un élément formant électrode (26), qui est en contact électrochimique avec un électrolyte (23),
   - une borne (31) pour établir un contact électrique avec l'élément formant électrode (26), et
   - un matériau céramique ou un matériau formé d'une résine (22), poreux, non perméable aux liquides, disposé sur l'électrolyte (23),

   caractérisée en ce qu'un autre matériau poreux (24) est prévu sur le matériau poreux (22) (figure 2).

9. Électrode selon la revendication 8, caractérisée en ce qu'il s'agit d'une électrode de référence, que l'électrolyte (23) est solide et contient des ions de l'élément formant électrode (26), que le matériau poreux (22) est une céramique ou un matériau formé d'une résine et que l'autre matériau poreux (24) est un matériau céramique résistant à la chaleur.

10. Électrode selon la revendication 8 et/ou 9, caractérisée en ce qu'une gaine (21) est disposée sur l'autre matériau poreux (24), cette gaine comportant des ouvertures permettant un contact de l'autre matériau poreux (24) avec la solution à mesurer.

FIG. 1

# FIG. 2

FIG. 3

FIG. 4

## FIG. 5

## FIG. 7

## FIG. 6

FIG. 8

FIG. 9

*FIG. 10*

*FIG. 11*

FIG. 12

FIG. 13

## FIG. 14

```
        ┌─────────────────┐
        │                 │
        └─────────────────┘
                 │
   ┌─────────────────────────┐
   │  DISSOLVED OXYGEN       │
   │  CONCENTRATION          │
   └─────────────────────────┘
                 │
           ╱ ABOVE ╲              NO
         ╱ PREDETERMINED REFERENCE ╲──────
         ╲    VALUE ?    ╱
           ╲          ╱
              │ YES
   ┌─────────────────────────┐
   │  DISSOLVED HYDROGEN     │
   │  CONCENTRATION          │
   └─────────────────────────┘
                 │
           ╱ BELOW  ╲              NO
         ╱ PREDETERMINED REFERENCE ╲──────
         ╲    VALUE ?    ╱
           ╲          ╱
              │ YES
   ┌─────────────────────────────────────┐
   │ OPEN HYDROGEN GAS INJECTION VALVE   │
   └─────────────────────────────────────┘
                 │
   ┌─────────────────────────┐
   │  DISSOLVED OXYGEN       │
   │  CONCENTRATION          │
   └─────────────────────────┘
                 │
           ╱ BELOW  ╲              NO
         ╱ PREDETERMINED REFERENCE ╲──────
         ╲    VALUE ?    ╱
           ╲          ╱
              │ YES
   ┌─────────────────────────┐
   │  DISSOLVED HYDROGEN     │
   │  CONCENTRATION          │
   └─────────────────────────┘
                 │
           ╱ ABOVE  ╲              NO
         ╱ PREDETERMINED REFERENCE ╲──────
         ╲    VALUE ?    ╱
           ╲          ╱
              │ YES

   ┌──────────────────────┐
   │ CLOSE HYDROGEN GAS   │
   │ INJECTION VALVE      │
   └──────────────────────┘
            │
   ┌─────────────────┐
   └─────────────────┘
```

29

# FIG. 15

# FIG. 16

# FIG. 17

FIG. 18(a)

FIG. 18(b)

# FIG. 19

# FIG. 20